# EUROPÄISCHE PATENTSCHRIFT

(11) **EP 3 380 625 B1**
(45) Veröffentlichungstag und Bekanntmachung des Hinweises auf die Patenterteilung: **05.08.2020**
(21) Anmeldenummer: 15808552.2
(22) Anmeldetag: 28.11.2015
(51) Int. Cl.: C12P 7/24, C12P 7/64, A23L 2/56, A23L 27/00, A23L 27/20, A23L 27/26

(54) **VERFAHREN ZUR HERSTELLUNG VERZWEIGTER ALDEHYDE**
METHOD FOR PRODUCING BRANCHED ALDEHYDES
PROCÉDÉ POUR LA PRÉPARATION D'ALDÉHYDES RAMIFIÉS

(43) Veröffentlichungstag der Anmeldung: 03.10.2018
(73) Patentinhaber: Symrise AG, 37603 Holzminden (DE)
(72) Erfinder: FRAATZ, Marco, 35392 Gießen (DE); ZORN, Holger, 35435 Wettenberg (DE); ROST, Johanna, 55262 Heidesheim (DE); GOLDMANN, Michael, 36145 Schackau (DE); GROSS, Egon, 37603 Holzminden (DE); LEY, Jakob, 37603 Holzminden (DE); GEISSLER, Katrin, 37574 Einbeck (DE); GEISSLER, Torsten, 37574 Einbeck (DE); BACKES, Michael, 37603 Holzminden (DE); HENTSCHEL, Fabia, 37603 Holzminden (DE); HILMER, Jens-Michael, 37603 Holzminden (DE)
(74) Vertreter: Eisenführ Speiser
(86) Internationale Anmeldenummer: PCT/EP2015/077992
(87) Internationale Veröffentlichungsnummer: WO 2017/088937

(56) Entgegenhaltungen:
- WO-A1-2012/025629
- TYRRELL D: "The fatty acid composition of some Entomophthoraceae", CANADIAN JOURNAL OF MICROBIOLOGY,, Bd. 17, 1971, Seiten 1115-1118, XP001320351,
- MIURA Y ET AL: "FATTY ACID AND LIPID COMPOSITIONS OF CONIDIOBOLUS", JOURNAL OF APPLIED BACTERIOLOGY, BLACKWELL PUBLISHING LTD., OXFORD, GB, Bd. 54, Nr. 1, 1983, Seiten 85-90, XP008065228, ISSN: 0021-8847
- TYRRELL D: "THE FATTY ACID COMPOSITOINS OF 17 ENTOMOPHTHORA ISOLATES", CANADIAN JOURNAL OF MICROBIOLOGY, NRC RESEARCH PRESS, CA, Bd. 13, 1967, Seiten 755-760, XP008082077, ISSN: 0008-4166
- HUANLU SONG ET AL: "Aroma extract dilution analysis of a beef flavouring prepared from flavour precursors and enzymatically hydrolysed beef", FLAVOUR AND FRAGRANCE JOURNAL., Bd. 23, Nr. 3, 2008, Seiten 185-193, XP055290064, GB ISSN: 0882-5734, DOI: 10.1002/ffj.1873

## Beschreibung

### GEBIET DER ERFINDUNG

Die Erfindung befindet sich auf dem Gebiet der Aromastoffe und betrifft ein neues Verfahren zur Herstellung verzweigter Aldehyde, insbesondere von 12-Methyltridecanal.

### STAND DER TECHNIK

Verzweigte Aldehyde stellen interessante Riech- und Aromastoffe dar, die in der Natur weit verbreitet sind. Insbesondere 12-Methyltridecanal ist ein wichtiger Baustein für authentisch schmeckende Rindaromen und ist derzeit nur durch chemische Synthese kommerziell herstellbar (vgl. Kerscher, et. al., J. Agric. Food Chem., 48(6), S. 2387-2390, 2000**).**

Basierend auf dem Herstellungsprozess kann es dadurch nicht als natürlich im Sinne der europäischen Aromengesetzgebung deklariert werden. Andererseits besteht im Markt ein besonderes Interesse an Aromen, die nicht nur als naturidentisch, sondern ausdrücklich als natürlich ausgelobt werden können; diese Deklarationsmöglichkeit stellt nicht zuletzt auch ein wichtiges Kauf- und Preisargument dar.

Die Aufgabe der vorliegenden Erfindung hat daher darin bestanden, ein Verfahren zur Herstellung zur Verfügung zu stellen, mit dem verzweigte Aldehyde im Allgemeinen und 12-Methyltridecanal im Besonderen auf biotechnologischem und daher als natürlich geltendem Wege hergestellt werden können.

### BESCHREIBUNG DER ERFINDUNG

Gegenstand der Erfindung ist ein Verfahren zur Herstellung verzweigter Aldehyde, vorzugsweise solcher, die 12 bis 18 Kohlenstoffatome umfassen und/oder eine Methylverzweigung aufweisen, insbesondere 12-Methyltridecanal, 12-Methyltetradecanal, 14-Methylpentadecanal, 16-Methyloctadecanal oder deren Gemische, umfassend die folgenden Schritte:
(a) Bereitstellen einer Kultur eines oder mehrerer Pilze der Gattung *Conidiobolus* ausgewählt aus der Species C. denaeosporus oder C. heterosporus, und Erzeugung von Biomasse mit einem Gehalt an verzweigten Carbonsäuren in freier und/oder gebundener Form als Glyceride;
(b) Extraktion der Biomasse aus Schritt (a) zur Erzeugung eines ersten Zwischenproduktes enthaltend freie und/oder gebundene Carbonsäuren;
(c) optional chemische, enzymatische oder mikrobielle Hydrolyse der gebundenen Carbonsäuren aus dem ersten Zwischenprodukt;
   und
(d) Behandlung des ersten Zwischenproduktes mit einem Reduktionsmittel chemischer Natur zur Überführung der freien und/oder gebundenen Carbonsäuren in die entsprechenden Alkohole sowie optionale Abtrennung eines oder mehrerer Alkohole von störenden Nebenkomponenten und Erzeugung des chemisch erzeugten zweiten Zwischenproduktes, welches diese Alkohole als Mischung oder in angereicherter Form enthält; und
(f) Behandlung des chemisch erzeugten zweiten Zwischenproduktes mit einem Oxidationsmittel chemischer Natur zur Überführung der freien und/oder gebundenen Alkohole in die entsprechenden Aldehyde;
   oder
(e) Behandlung des ersten Zwischenproduktes mit einem Reduktionsmittel biologischer Natur zur Überführung der freien und/oder gebundenen Carbonsäuren in die entsprechenden Aldehyde mit gleicher Anzahl an Kohlenstoffatomen im Vergleich zu den freien und/oder gebundenen Carbonsäuren oder in die korrespondierenden Aldehyde mit einer um eins verringerten Anzahl an Kohlenstoffatomen im Vergleich zu den freien und/oder gebundenen Carbonsäuren und Erzeugung des biologisch erzeugten zweiten Zwischenproduktes, welches diese Aldehyde enthält; sowie gegebenenfalls
(g) Entfernung von störenden Nebenkomponenten aus den Fraktionen erhältlich nach den Schritten (d) und/oder (e) und/oder (f).

Überraschenderweise wurde gefunden, dass einige der Species der bislang wenig beachteten Pilzgattung *Conidiobolus* ein Fettsäuremuster aufweisen, das diese Mikroorganismus als geeignete Ausgangsstoffe für die Herstellung verzweigter Aldehyde im Allgemeinen und 12-Methyltridecanal im Besonderen erscheinen lässt. Durch eine optimierte Behandlungsabfolge mit biologischen und/oder chemischen Reduktionsmitteln und chemischen Oxidationsmitteln lassen sich die Zielprodukte in ausreichender Ausbeute und hoher Reinheit gewinnen. Die Oxidationsprodukte stellen Mischungen verzweigter Aldehyde dar, die auch noch lineare Spezies enthalten. Diese Mischungen können unmittelbar eingesetzt werden, es ist jedoch ebenso möglich, durch gezielte Aufarbeitung das besonders begehrte Produkt 12-Methyltridecanal zu erhalten.

### ORGANISMEN

Bei den Organismen, die infolge des von ihnen produzierten Fettsäurespektrums als geeignete Bioreaktoren für die Gewinnung von verzweigten Fettsäuren der gewünschten Kettenlänge dienen, handelt es sich um um zwei Spezies der Gattung *Conidiobolus,* die hauptsächlich Saprobionten umfasst, die totes Pflanzenmaterial abbauen oder im Boden leben. Die beiden Spezies *C. denaeosporus* und *C. heterosporus* werden im Zusammenhang mit der Zersetzung der Blätter der chinesischen Ulme (*Ulmus parvifolia*) beschrieben. Taxonomisch werden die beiden Gattungen wie folgt eingeordnet:
Reich: Fungi
Subphylum: Entomophthoromycotina
Ordnung: Entomophthorales
Familie: Ancylistaceae

Übersichten zu diesen Organismen sind von D. Tyrrell in den Jahren 1968 bis 1976 veröffentlicht worden: "The fatty acid composition of some Entomophthoraceae. II. The occurrence of branched-chain fatty acids in Condiobolus denaesporus" Lipids 3 (4), S. 368-372 (1968**);** "Fatty acid composition of some Entomophthoraceae. III. " Can J Microbiol 17 (8), S. 1115-1118 (1971**);** und "The fatty acid composition of some Entomophthoraceae. IV. The occurrence of branched-chain fatty acids in Conidiobolus species". Can J Microbiol 22 (7), S. 1058-1060 (1976**).**

### KULTIVIERUNG

Die Kultivierung des oder der Pilzmyzele kann in an sich bekannter Weise unter Verwendung eines geeigneten Nährmediums in fester oder flüssiger Kultur erfolgen. Bewährt haben sich Agarplatten, die mit einer Nährlösung aus Malz- und Hefeextrakt hergestellt werden.

### EXTRAKTION

Nachdem die Kulturen eine ausreichende Menge an Biomasse hervorgebracht haben, wird diese - falls erforderlich - von der Nährlösung abgetrennt, beispielsweise durch Zentrifugieren, Filtration oder andere geeignete Trennverfahren. Es schließt sich die Extraktion der Carbonsäuren in ihrer freien oder gebundenen Form an. Feste Kulturen, wie sie beispielsweise nach einer Gefriertrocknung erhalten werden, können direkt extrahiert werden.

Sofern des Weiteren auf Extrakte Bezug genommen worden ist, kann deren Herstellung in an sich bekannter Weise erfolgen, d.h. beispielsweise durch wässrigen, alkoholischen oder wässrig-alkoholischen Auszug der Kulturen selbst oder der daraus gewonnenen Trockenmasse. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden.

Besonders bevorzugt ist die Extraktion mit Methanol, Ethanol, Pentan, Hexan, Heptan, Aceton, Propylenglykole, Polyethylenglykole sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt.

Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion der Biotrockenmasse liegen im Bereich von 3 bis 15, insbesondere 6 bis 10 Gew.-%. Die vorliegende Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.-% aufweisen, können als solche eingesetzt werden, es ist jedoch ebenfalls möglich, das Lösungsmittel durch Trocknung, insbesondere durch Sprüh- oder Gefriertrocknung vollständig zu entfernen.

Die Extrakte, die im Weiteren auch als erstes Zwischenprodukt bezeichnet werden, können sowohl als wässrige und/oder in organischen Solventien gelöste Zubereitungen als auch sprüh- bzw. gefriergetrocknete, wasserfreie Feststoffe vorliegen. Als organische Lösungsmittel kommen in diesem Zusammenhang beispielsweise die aliphatischen oder verzweigten Alkohole mit 1 bis 6 Kohlenstoffatomen (z.B. Ethanol, 2-Methoxy-2-methylpropan), Ketone (z.B. Aceton), Halogenkohlenwasserstoffe (z.B. Chloroform oder Methylenchlorid), niedere Ester oder Polyole (z.B. Glycerin oder Glycole) in Frage.

### HYDROLYSE

Im Bedarfsfall können die Extrakte vor der Reduktion einer Hydrolyse unterworfen werden, um den Anteil an Fettsäuren, der als Glycerid gebunden vorliegt, freizusetzen. Die Hydrolyse kann dabei chemisch erfolgen, vorzugsweise wird man jedoch eine biologische Alternative wählen. Diese kann sowohl mikrobiell, d.h. mittels lebender Mikroorganismen, als auch enzymatisch erfolgen, da diese schonender verlaufen und zudem regulatorische Vorteile bieten. Für die biologische Alternative sind Hydrolasen beispielsweise aus der Gattung *Candida* geeignet, insbesondere aus *Candida rugosa* oder *Candida cylindracea.*

### REDUKTION

Nach der Extraktion wird als erstes Zwischenprodukt eine Mischung aus unterschiedlichen linearen und verzweigten, gesättigten und ungesättigten Fettsäuren erhalten, die auch als Glyceride gebunden vorliegen können. Eine solche Mischung kann beispielsweise die folgenden Vertreter umfassen: Tridecansäure, 12-Methyltridecansäure, Tetradecansäure, 12-Methyltetradecansäure, Pentadecansäure, 14-Methylpentadecansäure, Hexadecansäure, Hexadecen(9)säure, 15-Methylhexadecansäure, Heptadecansäure, Elaidinsäure, Ölsäure und Arachidonsäure.

Die Mischung der Fettsäuren- bzw. Fettsäureglyceride wird nun einer Reduktion unterzogen. Diese kann wie auch die Hydrolyse chemisch erfolgen, vorzugsweise wird man jedoch eine biologische Alternative wählen, da diese dann zu einem im Sinne der europäischen Aromengesetzgebung als natürlich geltendem Produktionsprozess führt. Die bevorzugte biologische Alternative kann dabei sowohl mit Enzymen als auch mit Zelllysaten, -extrakten oder auch mit ganzen Zellen durchgeführt werden, die die für die Reduktion benötigte enzymatische Aktivität aufweisen. Nach diesem Schritt können die Reaktionsprodukte mit dem Fachmann bekannten Extraktionsverfahren aus dem Reaktionsgemisch extrahiert werden. Nach Entfernung des Extraktionsmittels kann dann das erhaltene Konzentrat in der gewünschten Konzentration mit dem Lösemittel der Wahl aufgenommen werden.

Die chemische Reduktion der Mischung der Fettsäuren bzw. Fettsäureglyceride kann mittels Natriumborhydrid erfolgen, vorzugsweise sollte aber Lithiumalanat zum Einsatz kommen. Im Zuge dieser Umsetzung werden die Säure- oder Esterfunktionen zu Hydroxylgruppen reduziert; das zweite, in diesem Falle chemische Zwischenprodukt, enthält somit ganz oder zumindest überwiegend die entsprechenden Alkohole der linearen und verzweigten Carbonsäuren. Innerhalb von Reaktionszeiten von etwa 3 Stunden werden Ausbeuten um die 85 bis 95 % der Theorie erzielt, die erhöht werden können, wenn sich an die Reduktion noch ein destillativer Aufarbeitungsschritt anschließt, beispielsweise eine Kugelrohrdestillation. Bezogen auf die Menge an dem besonders begehrten Produkt 12-Methytridecanol als Reduktionsprodukt der 12-Methytridecansäure liegen die Ausbeute bei etwa 25 bis 35 % bezogen auf die Gesamtmenge an Reduktionsprodukten. Bei der Reduktion kommt es als Nebenreaktion auch zur Absättigung im Gemisch enthaltener ungesättigter Spezies.

Reduktionsverfahren unter Einsatz von Hydriden gehören zu den Standardreaktionen des präparativen organischen Chemikers und stellen Lehrbuchwissen dar, das daher keiner weiteren umfassenden Erläuterung bedarf. Vorzugsweise wird die Reduktion bei Temperaturen im Bereich von 15 bis 25 °C durchgeführt, wobei erwartungsgemäß ein vorübergehender Temperaturanstieg zu Beginn der Reaktion auftritt.

### OXIDATION

Das zweite chemische Zwischenprodukt dient als Ausgangsmaterial für die nachfolgende Oxidation, bei der die Alkohole in die entsprechenden Aldehyde überführt werden. Als Oxidationsmittel wird vorzugsweise TEMPO eingesetzt. TEMPO steht für 2,2,6,6-Tetramethylpiperidinyloxyl.

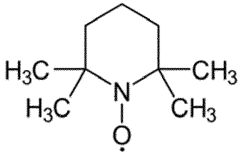

Dieses Radikal ist thermodynamisch zwar nicht stabil, doch rührt seine vergleichsweise hohe Persistenz von Substituenten her, welche die Lebensdauer durch sterische Effekte beeinflussen. Die Substituenten befinden sich in Nachbarschaft zum Radikalelektron, sodass es in sauerstofffreier Lösung eine mittlere Lebensdauer von einer Minute besitzt. Die Verwendung von TEMPO zusammen mit einem geeigneten Co-Oxidationsmittel zur Zweiphasenoxidation von Alkoholen wird von Anelli et al. in J Organic Chemistry. 52, S. 2559-2562 (1987**)** beschrieben ("Anelli-Oxidation") und beispielsweise in Organic Syntheses, Coll. Vol. 8, S. 367 (1993**);** Vol. 69 S.212 (1990**)** angewendet.

Im Sinne der vorliegenden Erfindung hat es sich als vorteilhaft erwiesen, TEMPO zusammen mit zwei Co-Oxidationsmitteln, nämlich einem Alkalibromid und einem Alkalihypochlorit einzusetzen. Bevorzugt ist hier die Kombination von Kaliumbromid und Natriumhypochlorit. Dabei hat sich ein molares Einsatzverhältnis von TEMPO, Alkalibromid und Alkalihypochlorit von etwa 1:(2 bis 10):(10 bis 40) besonders, insbesondere ein molares Einsatzverhältnis von TEMPO, Alkalibromid und Alkalihypochlorit von etwa 1:(2 bis 10):(10 bis 40) und insbesondere von etwa 1:(4 bis 8):(15 bis 30) bewährt. Da die Reaktion stark exotherm ist, empfiehlt es sich, die Reaktionstemperatur im Bereich von etwa -5 bis +10 °C zu halten.

Unter diesen Bedingungen werden Ausbeuten im Bereich von 85 bis 97 % bezogen auf die eingesetzten Alkohole erhalten. Bezogen auf das bevorzugte Zielprodukt 12-Methyltridecanal beträgt die Ausbeute 30 bis 35 %, wobei eine Reinheit von ca. 95 % erzielt wird.

Auch im Fall der Oxidation kann die Reinheit weiter verbessert werden, indem man die Produkte einer destillativen Aufarbeitung unterwirft, wie beispielsweise einer Kugelrohrdestillation oder unter Einsatz eines Spaltrohrs.

Bei der Wahl eines enzymatischen Prozesses zur biologischen Reduktion können Enzyme wie kommerziell erhältliche Aldehyd-Dehydrogenasen (ALDH, EC 1.2.1.x) als auch Carbonsäurereduktasen wie z.B. aus *Norcardia* sp. (Aimin He, Tao Li, Lacy Daniels, lan Fotheringham, John P. N. Rosazza Appl Environ Microbiol. 2004 Mar**;** 70(3): 1874-1881**)** und/ oder *Mycobacterium marinum* (M. Kalim Akhtar, Nicholas J. Turner, Patrik R. Jones Proc Natl Acad Sei USA. 2013 Jan 2**;** 110(1): 87-92**)** verwendet werden. Carbonsäurereduktasen können durch homologe Expression in *Nocardia sp.* oder *Mycobacterium sp.* insbesondere *Nocardia iowe*nsis als auch heterolog durch rekombinante Expression in geeigneten Wirtsorganismen insbesondere *E. coli* erhalten werden, bevorzugt aber auch durch Anzucht von *Nocardia iowe*nsis erhalten werden, der die Enzyme als sog. Wildtypstamm ebenfalls exprimiert. Dabei ist es für die prinzipielle Umsetzung irrelevant, ob die Enzyme in gereinigter Form, nur teilweise aufkonzentriert, in einem Zellrohextrakt oder in nativen oder rekombinanten Zellen vorliegen.

### GEWERBLICHE ANWENDBARKEIT

Durch das erfindungsgemäße Verfahren kann ein Konzentrat methylverzweigter Aldehyde mit 12 bis 18 Kohlenstoffatomen, welches diese in Mengen von 10 bis 100, vorzugsweise 25 bis 95 und insbesondere etwa 40 bis etwa 60 Gew.-% enthält, enthalten werden. Die Konzentrate können direkt auf Basis des chemisch erzeugten zweiten Zwischenproduktes (f) sowie des biologisch erzeugten zweiten Zwischenproduktes (e) oder der vollständig oder teilweise gereinigten Produkte (g) durch geeignete, vorzugsweise schonende Trockenverfahren erhalten werden, als da beispielsweise Sprüh- oder insbesondere Gefriertrocknung sind. Die Differenz zu 100 Gew.-% können dabei Träger und/oder andere Aromastoffe ausmachen. Alternativ können auch konzentrierte wässrige Lösungen in den Handel kommen.

Die nach dem erfindungsgemäßen Verfahren hergestellten Konzentrate oder alternativ der unmittelbaren Verfahrensprodukte wie oben beschrieben können als Aromakomponenten, insbesondere als Aromakomponenten für Nahrungsmittel, verwendet werden um diesen einen Rindsgeschmack zu verleihen.

### BEISPIELE

### BEISPIEL 1

### Kultivierung der Pilzstämme

### EINGESETZTE PILZSTÄMME

In der nachfolgenden **Tabelle 1** wird eine Übersicht über die untersuchten Pilze, ihre genaue Bezeichnung, Nummer der Stammsammlung (CBS-Nummer), das Isolationssubstrat sowie den Isolationsort gegeben.

**Tabelle 1**

| Untersuchte Pilze | | | |
|---|---|---|---|
| **Organismus** | **CBS-Nummer** | **Isolationssubstrat, -ort** | **Abkürzung** |
| *Conidiobolus denaeosporus* | 137.57 | zersetzte Blätter von *Ulmus parvifolia,* (US) | CDE |
| *Conidiobolus heterosporus* | 333.74 | landwirtschaftlicher Boden (NL) | CHET-N |
| *Conidiobolus heterosporus* | 138.57 | Waldblätterstreu (US) | CHET-U |

### Kultivierung auf festem Medium

Die Pilzstämme wurden auf Malzextrakt-Agarplatten kultiviert. Zur Herstellung der Platten wurden die Medienbestandteile eingewogen (Tabelle 2), mit destilliertem Wasser ad 1 Liter aufgefüllt und autoklaviert (120 °C, 20 min). Auf die Agarplatten wurde mittig ein 1 cm² großes, mit Myzel bewachsenes Agarstück aus dem vitalen Randbereich einer Plattenkultur positioniert, die Petrischalen mit Parafilm verschlossen und anschließend bei 24 °C im Brutschrank mit oder vorzugsweise ohne Licht inkubiert. Nach ca. 5 Tagen (CHET-N) bzw. 14 Tagen (CHET-U und CDE) wurden von den zu etwa dreiviertel bewachsenen Platten Vorkulturen angeimpft. Parallel wurden fortlaufend weitere Agarplatten für die nächsten Vorkulturen nach dem gleichen Prinzip beimpft. **Tabelle 2** zeigt die Zusammensetzung des Malzextrakt-Hefeextrakt-Agars zur Anzucht von *Conidiobolus-Stämmen:*

**Tabelle 2**

| **Zusammensetzung Agar** | |
|---|---|
| **Medienbestandteil** | **Konzentration [g L⁻¹]** |
| Agar | 15 |
| Malzextrakt | 30 |
| Hefeextrakt | 3 |

Für die submers geführten Vorkulturen wurde ein komplexes Nährmedium verwendet, das in **Tabelle 3** wiedergegeben ist:

**Tabelle 3**

| Zusammensetzung des Standard-Komplexmediums | |
|---|---|
| **Medienbestandteil** | **Konzentration [g L⁻¹]** |
| Malzextrakt | 30 |
| Hefeextrakt | 3 |

Für die Kultivierung in Submerskultur wurde unter sterilen Bedingungen ein 1 cm² großes Agarstück des äußeren Myzelrasens in einen mit 100 mL Nährmedium befüllten 250-mL-Erlenmeyerkolben überführt und mit einem Ultraturrax-Dispergierstab behandelt (30 s, 9.800 U min⁻¹). Die Vorkulturen wurden bei 24 °C unter Lichtausschluss im Inkubationsschüttler (Multitron, Infors, Einsbach; 24 °C, 150 U min⁻¹, Auslenkung 25 mm) inkubiert.

Hauptkultur: Für die Anzucht der Hauptkulturen wurde die Vorkultur unter sterilen Bedingungen mit einem Ultraturrax-Dispergierstab behandelt (30 s, 9.800 U min⁻¹). 20 mL dieser Suspension wurden zu 200 mL frischem Nährmedium in einem 500-mL-Erlenmeyerkolben hinzugefügt. Die Kultivierung erfolgte bei 24 °C unter Lichtausschluss im Inkubationsschüttler (24 °C, 150 U min⁻¹, Auslenkung 25 mm).

### BEISPIEL 2

### Zellernte und Fettextraktion

Die Hauptkultur wurde am Ende der Kultivierungsdauer durch ein Filterpapier (Rückhaltebereich 8 - 12 µm) filtriert. Der Rückstand wurde mit etwa 200 mL 4 M HCl versetzt, bis zum Sieden erhitzt und für 30 min in der Siedehitze aufgeschlossen. Die Lösung wurde anschließend im noch heißen Zustand durch ein Faltenfilter filtriert und so lange mit heißem Wasser gewaschen, bis das Waschwasser neutral war. Nach 1 - 2 h Trocknen im Trockenschrank (100 °C) wurde das Filterpapier an einer Soxhlet-Apparatur für 4 h mit Petroleumbenzin (Siedebereich 40 -60 °C) extrahiert. Nach Entfernen des Lösungsmittels mittels Rotationsverdampfer wurde der Fettgehalt gravimetrisch bestimmt.

Alternativ dazu kann die Hauptkultur am Ende der Kultivierungsdauer durch ein Filterpapier (Rückhaltebereich 8 - 12 µm) filtriert und anschließend lyophilisiert werden. Das Lyophilisat wurde dann mit gereinigtem Sand verrieben, 3 mal mit je ca. 75ml Hexan versetzt und mit 40 - 120 bar Druck in einem SpeedExtactor der Firma Büchi extrahiert. Nach Entfernen des Lösungsmittels mittels Rotationsverdampfer wurde der Fettgehalt gravimetrisch bestimmt.

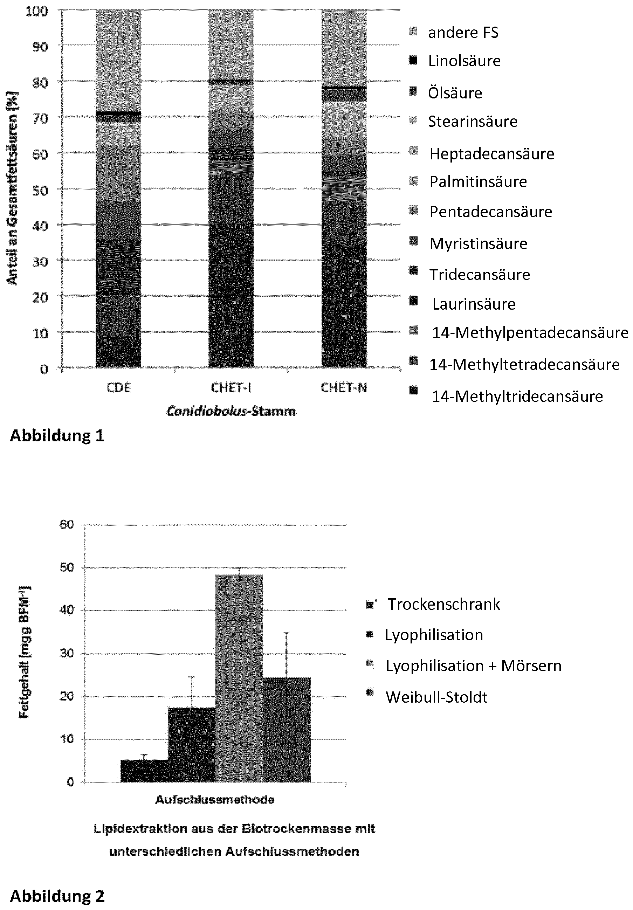

Zur Identifikation der am besten geeigneten Aufarbeitungsmethode der fermentativ hergestellten Biomasse wurden verschiedene Ansätze untersucht. Hierbei wurde die Lyophilisation mit anschließender Feinzerteilung / -vermahlung als die am besten geeignete Methode identifiziert. **Abbildung 1** zeigt die Anteile der Fettsäuren am Gesamtfettsäurengehalt der umgeesterten Lipidextrakte verschiedener *Conidiobolus*-Stämme; alle Fettsäuren wurden als Fettsäuremethylester gaschromatographisch bestimmt. Der Fettgehalt in Abhängigkeit der Aufschlussmethode ist in **Abbildung 2** wiedergegeben.

### BEISPIEL 3

### Enzymatische Hydrolyse mittels Lipase

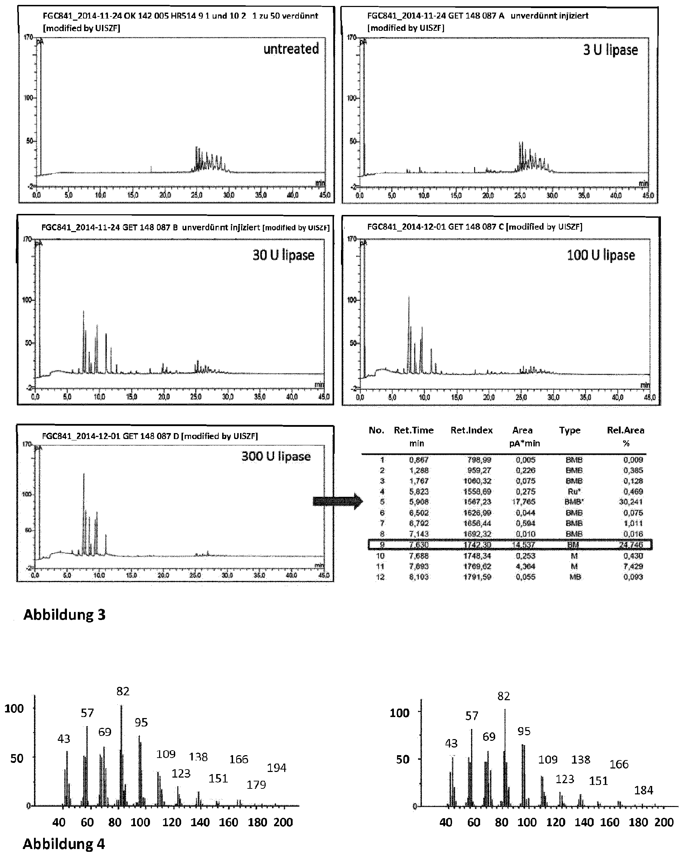

Von dem nach der Extraktion der Biomasse erhaltenen Lipidextrakt wurden etwa 50 mg mit 10 - 100 µl einer Lipase-Enzymlösung der "Lipase AY" von Amano (aus *Candida cylindracea*) unterschiedlicher Konzentrationen mit 100 - 190 µL Phosphat-Puffer (200 mM, pH 7,6) und 100 µL Wasser versetzt und über Nacht in einem Thermoblock (Eppendorf) inkubiert (45 °C, 800 U min⁻¹). Zur Herstellung des Puffers wurde eine 0,2 M Na₂HPO₄ Lösung hergestellt und so mit einer 0,2 M KH₂PO₄ Lösung gemischt, dass sich ein pH-Wert von 7,0 einstellt. Die untersuchten Konzentrationsverhältnisse sind in **Tabelle 4** wiedergegeben. Anschließend wurden die Proben mit 2 mL Hexan ausgeschüttelt und die obere (organische) Phase in Glasfläschchen umgefüllt um die Fettsäureverteilung mittels GC (ohne vorhergehende Methylierung) zu bestimmen **(****Abbildung 3****).** In der unbehandelten Fettfraktion lagen keine freien Fettsäuren vor. Durch Hydrolyse mit der Lipase wurden Fettsäuren freigesetzt, stark abhängig von der eingesetzten Enzymmenge (mit 3 U Enzym wird nur sehr wenig freigesetzt, mit 30 U dagegen deutlich mehr). Insgesamt wurden 14 % 12-Methyltridecansäure gefunden. Bei einer Wiederholung der Versuche mit jeweils 100 U und 300 U Enzym wurden insgesamt 25 Gew.-% 12-Methyltridecansäure nachgewiesen.

**Tabelle 4**

| Reaktionsansätze zur Hydrolyse des Fettextraktes | | | | |
|---|---|---|---|---|
| | **A** | **B** | **C** | **D** |
| Fettfraktion | 50 mg | 50,4 mg | 51 mg | 51,3 mg |
| Lipase-Lösung (10 mg/ml) | 10 µl | 100 µl | | |
| Lipase-Lösung (100 mg/ml) | | | 20 µl | 100 µl |
| Lipasemenge (U Lipase) | 3 | 30 | 100 | 300 |
| Phosphat-Puffer (200 mM), pH 7 | 190 µl | 100 µl | 180 µl | 100 µl |
| H₂O | 100 µl | 100 µl | 100 µl | 100 µl |

### BEISPIEL 4

### Umsetzung von 12-Methyltridecansäure mit α-Dioxygenase

Die Produktion der α-Dioxygenase erfolgte nach Literaturangaben ausgehend von dem in der Patentanmeldung WO2012025629A1 näher beschriebenen Verfahren. Zur Zelltransformation wurde 1 µL des Plasmids zu kompetenten *Escherichia coli*-Zellen [BL21(DE3), Novagen] hinzu pipettiert. Nach einer Abkühlphase (30 min auf Eis) wurden die Proben für 2 min im Wasserbad (42 °C) erwärmt und erneut abgekühlt. Zur Kultivierung wurden 150 µL LB-Kanamycin-Medium **(Tabelle 5)** hinzugefügt und 1 h inkubiert (37 °C, 200 U min⁻¹, Auslenkung 25 mm). Die Zellsuspension wurde auf vorgetrocknete und akklimatisierte LB-Kanamycin-Agar-Platten aufgebracht und über Nacht bei 37 °C inkubiert.

**Tabelle 5**

| Zusammensetzung des LB-Kanamycin-Mediums | |
|---|---|
| **Menge** | **Komponente** |
| 10 g L⁻¹ | Trypton |
| 5 g L⁻¹ | Hefeextrakt |
| 10 g L⁻¹ | NaCl |
| 25 mg L⁻¹ | Kanamycin (nicht autoklaviert, sondern steril filtriert hinzugegeben) |

Für die Umsetzungen mit der α-Dioxygenase wurde dieses Enzym entsprechend den Angaben von WO 2012 025629 A1 in *E*. *coli*-Zellen [BL21(DE3)] in LB-Medium exprimiert. Von dem Bakterienrasen wurde mittels Impföse ein ca. 0,5 cm langer Abstrich entnommen, in 3 mL LB-Kanamycin-Medium überführt und bis zu einer OD₆₀₀ zwischen 0,5 - 0,8 im Schüttler inkubiert (37 °C, 150 U min⁻¹, Auslenkung 25 mm). Isopropyl-β-D-thiogalactopyranosid (IPTG) wurde hinzugefügt (0,5 mM) und die Probe weitere 16 - 18 h im Schüttler (24 °C, 150 U/min, Auslenkung 25 mm) inkubiert. Die Probe wurde zentrifugiert (4.000 U/min, 3.724 x g, 10 min, 4 °C), der Überstand verworfen und das Zellpellet eingefroren (-20 °C).

Wie oben beschrieben hergestellte eingefrorene Zellpellets wurden in Phosphatpuffer (200 mM, pH 7,6) aufgenommen, im gleichen Puffer gewaschen, zentrifugiert und in 2 mL Phosphatpuffer, welchem 0,5% Glucose-Monohydrat zugesetzt wurden, resuspendiert. Zu 1 mL der Zellsuspension wurden 50 µL 12-Methyltridecansäure- (7,5 mg mL⁻¹ in DMSO) hinzugegeben. Die Probe wurde für 1 h bei 37 °C inkubiert (150 U min⁻¹, Auslenkung 25 mm) und anschließend mit 1 mL Heptan extrahiert. Nach dem Trocknen der organischen Phase über Natriumsulfat wurde diese gaschromatographisch untersucht.

### BEISPIEL 5

### Umsetzung des Lipidextrakts mit α-Dioxygenase

Die Umsetzung erfolgte vergleichbar zu dem Beispiel 4, bei dem eine Standardlösung mit 12-Methyltridecansäure verwendet wurde. Zu 2 ml der in Phosphatpuffer resuspendierten E. coli-Zellen wurden 10µl des Fettextraktes einer Pilzkultivierung aus Beispiel 2 hinzugegeben wie in Beispiel 4 extrahiert und anschließend gaschromatografphsch untersucht.

### BEISPIEL 6

### Gleichzeitige Umsetzung mit Lipase und mit α-Dioxygenase

145 mg eines Lipidextrakts von *C*. *denaeosporus* aus Beispiel 2 wurden in ein 10-mL-Vial eingewogen und mit 200 µL Lipase-Enzymlösung aus Beispiel 3, 200 µL Phosphat-Puffer (200 mM, pH 7,6) und 200 µL Wasser versetzt. 200 mg der α-Dioxygenase produzierenden *E*. *coli*-Zellen aus Beispiel 4 wurden mit Phosphatpuffer (200 mM; pH 7,6) gewaschen und in 4 mL Phosphatpuffer, welcher 0,5% Glucose- Monohydrat enthielt, resuspendiert und in das vorbereitete 10 mL Vial gegeben. Der Reaktionsansatz wurde über Nacht inkubiert (22 h, 24 °C, 150 U min⁻¹, Auslenkung 25 mm). Das umgesetzte Fett wurde 3mal mit je 2 mL Heptan extrahiert und die organischen Phasen vereint. Nach Verdünnung (1:50) wurden die Proben gaschromatographisch analysiert.

### BEISPIEL 7

### Umsetzung mit Aldehyd-Dehydrogenase (ALDH)

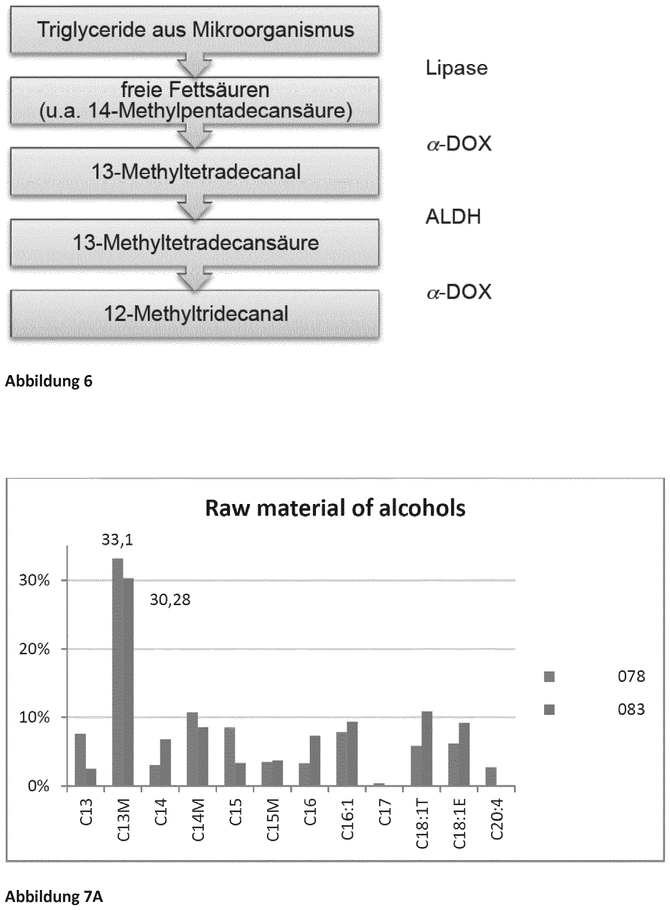

Zur Umsetzung mit Aldehyd-Dehydrogenase (ALDH aus *Saccharomyces cerevisiae,* 10,5 U mg⁻¹ Protein) wurden ca. 15 mg 12-Methyltridecansäure mit 500 µL Triton-X-100-Lösung (2,2 g L⁻¹) emulgiert. Die Umsetzung erfolgte gemäß Sigma *Quality Control Testl* in Anlehnung an Bostian et. al, (1978) Biochemical Journal 173, 773-786**.** Die Proben wurden nach Enzymzugabe im Schüttler inkubiert (30 min, 24 °C, 150 U min⁻¹, Auslenkung 25 mm) und anschließend 2mal mit je 1 mL Pentan/Diethylether (1:1,12, v/v) ausgeschüttelt. Vor der gaschromatographischen Analyse wurde die Probe mit 50 µL internem Standard (750 mg L⁻¹ Thymol) versetzt. Alternativ dazu wurde gemäß **Abbildung 6** in einem weiteren Experiment eine Reduktion mit einer α-Dioxygenase durchgeführt.

### BEISPIEL 8

### Reduktion von 12-Methlytridecansäure mit Karbonsäurereduktasen

Die Proteinexpression der Nsp-CAR wie auch der MmFad9 wurde in rekombinanten *E.coli* BL21 (DE3) Zellen durchgeführt. Hierzu wurden 50ml LB-Medium mit 30µg Kanamycin/ml aus einer Stammkonserve angeimpft und bei 37°C und 130 Upm so lange inkubiert, bis eine OD₆₀₀ von etwa 0,6 - 0,8 erreicht wurde. Beim Erreichen dieses Wertes wurde durch Zugabe von 1mM IPTG induziert und die Kulturen über Nacht bei Raumtemperatur weiter angezogen. Die so erhaltene Biomasse wurde bei 10.000 Upm für 10 Minuten zentrifugiert und das Zellpellet bis zur weiteren Verwendung bei -20°C gelagert.

Zur Proteingewinnung wurden die bei -20°C gelagerten Pellets aufgetaut und mit 4 ml B-PER Reagenz pro g Pellet resuspendiert. Nach der Zugabe von 50µg Lysozym/ml und 2,5 U DNase/ml wurden die Proben für 15 Minuten bei Raumtemperatur inkubiert, anschließend für 10 Minuten bei 10.000 Upm und 4°C zentrifugiert, um so den Gesamtproteinextrakt zu erhalten. Nach der folgenden Aufreinigung der Enzyme mittels HisPur Ni-NTA Säulen wurden die Proteinkonzentrationen der aufgereinigten Enzyme nach Bradford bestimmt (9,92 mg/ml für die Nsp-CAR und 26,43 mg/ml für die MmFad9).

Der Enzymassay wurde mit 50mM Tris-HCI Puffer (pH = 7,5), 20mM Substrat, 10 mM ATP, 10 mM NADPH, 100 mM MgCl₂ und 1 mg des aufgereinigten Enzyms so durchgeführt, dass das Gesamtvolumen 1,1 ml betrug. Nach einer Inkubationszeit von 24 h bei Raumtemperatur wurde die wässrige Mischung 3mal jeweils 1:1 mit Hexan ausgeschüttelt und die obere, organische Phase in ein GC-Fläschchen überführt. Die erhaltenen Resultate sind in **Tabelle 6** zusammengestellt. In **Abbildung 4****¹** sind die Massenspektren des Standards von 12-Methyltridecanal (rechter Teil) als auch nach der Reduktion mit der Nsp-CAR dargestellt (linker Teil).

**Tabelle 6**

| 12-Methyltridecansäure und 12-Methyltridecanalgehalte der enzymatischen Umsetzungen | | | | | | |
|---|---|---|---|---|---|---|
| | **12-Methyltridecanal [µg/ml]** | | | **12-Methyltridecansäure [µg/ml]** | | |
| | Wdh.1 | Wdh.2 | Wdh.3 | Wdh.1 | Wdh.2 | Wdh.3 |
| MmFad9; 1. Extraktion | 1,38 | 1,755 | 1,98 | 3105 | 3382,5 | 3333 |
| MmFad9; 2. Extraktion | 0,2125 | 0,4 | 0,3625 | 153,75 | 192,5 | 197,5 |
| MmFad9; 3. Extraktion | - | - | - | 12 | 20 | 22 |
| Nsp-CAR; 1. Extraktion | 1,475 | 1,7125 | 1,8625 | 2850 | 2975 | 2975 |
| Nsp-CAR; 2. Extraktion | 0,3 | 0,475 | 0,3 | 153,75 | 162,5 | 160 |
| Nsp-CAR; 3. Extraktion | - | - | - | 20 | 22 | 23 |

¹Abbildung 4: links: Massenspektrum von 12-Methyltridecanal generiert aus der homologen Säure durch Reduktion mittels CAR; rechts: Massenspektrum des Standards von 12-Methytridecanal

### BEISPIEL 9

### Reduktion von 12-Methyltridecansäure in einer Biotransformation (mit lebenden E. coli BL21-Zellen, die entweder Nsp-CAR oder MmFad9 exprimieren können)

Dadurch, dass von Akhtar et .al. (P Natl Acad Sci USA 2013;110: 87-92**)** beschrieben wurde, dass die katalytische Aktivität von Reduktasen durch die Anwesenheit des Proteins BsSfp positiv beeinflusst werden kann, wird ein entsprechender *E. coli* Stamm hierfür in LB Medium parallel bei 37 °C und 150 Upm über Nacht angezogen und zum Biotransformationsansatz hinzugegeben.

Von den Reduktase und dem Hilfsenzym exprimierenden *E. coli* Zellen wurden zunächst Kulturen angezogen, sodass diese eine OD von etwa 2 erreichten. Die Biotransformationsansätze wurden dann so angesetzt, dass pro Ansatz jede Reduktase einmal mit bzw. ohne Hilfsprotein sowie je ein Ansatz als Positivkontrolle vorlag, bei dem jeder Reduktase 100 µl einer Benzoesäurelösung zugegeben wurde. Neben den Volumina von jeweils etwa 1 ml, die für die exprimierenden *E.coli* Stämme benötigt wurden, wurde jedem Ansatz noch 5 µl IPTG (Endkonzentration im Ansatz von 1mM), 100 µl einer ethanolischen 12-Methyltridecansäurelösung (Endkonzentration im Ansatz von 2mM) sowie etwa 3 bis 4 ml frisches LB-Medium zugegeben, sodass die Ansätze, nach Fertigstellung eine OD von ca. 0,5 in einem Endvolumen 5 ml hatten. Die so hergestellten Ansätze wurden für 24 h bei Raum-temperatur bei einer Schüttelfrequenz von 150 Upm als Doppelbestimmungen kultiviert. Anschließend wurden die Ansätze je 2mal mit Hexan im Verhältnis 1:1 extrahiert. Die Resultate aus den Biotransformationen sind in **Tabelle 7** zusammen gestellt.

**Tabelle 7**

| Resultate der Biotransformationen zur Bildung von 12-Methyltridecanal | | | | | | |
|---|---|---|---|---|---|---|
| **Enzym** | **12-Methyltridecanal µg/ml** | | **12-Methyltridecansäure µg/ml** | | **12-Methyltridecanol µg/ml** | |
| Nsp-CAR | 0,77 | 0,75 | 5,97 | 5,80 | 24,7 | 22,5 |
| Nsp-CAR+BsSfp | 0,58 | 0,51 | 13,09 | 12,68 | 11,1 | 10,5 |
| MmFad9 | 0,31 | 0,31 | 58,32 | 63,51 | 2,5 | 2,8 |
| MmFad9+BsSfp | 0,28 | 0,33 | 62,33 | 64,93 | 0,9 | 0,9 |

### BEISPIEL 10

### Hydrolyse einer Fettfraktion von Conidiobolus heterosporus mittels Lipase und anschließende Umsetzung mit den Carbonsäurereduktasen MmFad9 bzw. Nsp-CAR zur Bildung von 12-Methyltridecanal

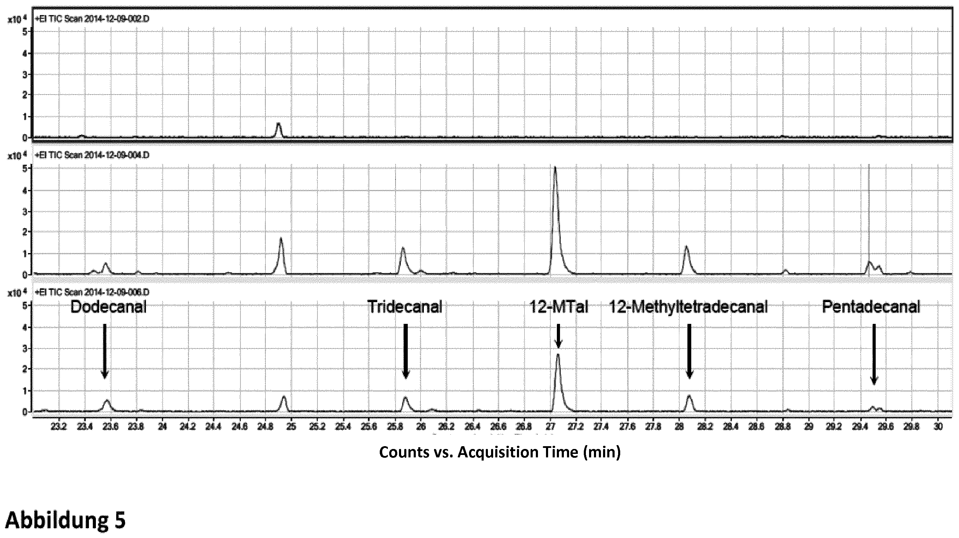

Die Hydrolyse wurde mit vereinigten Lipidextrakten, wie sie aus der Beschreibung in Beispiel 2 erhalten werden können, gemäß der Beschreibung aus Beispiel 3 durchgeführt. 150 mg des so hydrolysierten Fettextraktes wurden mit 1010 µl eines Reaktionsgemisches, wie es in **Tabelle 8** dargestellt ist, versetzt. Mittels MS/MS Analysen, dargestellt in **Abbildung 5****²**, konnte gezeigt werden, dass sowohl mit dem hydrolysierten als auch mit dem nicht hydrolysierten Fettextrakt 12-Methyltridecanal identifiziert werden konnte.

**Tabelle 8**

| Zusammensetzung der Reaktionsgemische zur Reduktion des hydrolysierten Fettextraktes | |
|---|---|
| **Substanz** | **Eingesetzte Menge** |
| CAR-Proteinlösung aus Beispiel 8 | 900 µL |
| ATP-Dinatriumsalz | 1mM |
| NADPH-Tetranatriumsalz | 1mM |
| MgCl2 * 6 H2O | 10mM |
| Endvolumen | 1,010 ml |

² Abbildung 5: oben: MS/MS-Chromatogramm (TIC, Scan in Q1) des Blindwertes (hitzeinaktivierte CAR) der enzymatischen Umsetzung des Lipidextraktes; mittig: Umsetzung des Lipidextraktes ohne Zusatz von Lipase; unten: Umsetzung des Lipidextraktes mit Lipase und CAR

### BEISPIEL 11

### Reduktion eines Fettextraktes von Conidiobolus heterosporus mit lebenden Zellen von Nocardia iowensis zur Herstellung von 12-Methytridecanal

Aus einer Cryokonserve von *Nocardia iowensis* (DSM 45197) wurde 1 mL entnommen und in einen 100ml Kolben überführt, der 10 ml LB-Medium und 0,05% Tween 80 enthielt. Der Kolben wurde für ca. 72 h bei 28 °C und 130 rpm bei einer Schüttelamplitude von 50 mm kultiviert. Im Anschluss daran wurde aus der gut bewachsenen Kultur 1 ml entnommen und in einen 100 ml Erlenmeyerkolben mit ebenfalls 10 ml desselben, frischen Mediums überführt. Analog zu T. Li et. Al. (J Bacteriol. Jun 1997; 179(11): 3482-3487**)** wurden dann mit 5 mg Benzoesäure /ml induziert und der Kolben für weitere 24 h bei 28 °C und 130 rpm inkubiert. Im Anschluss daran wurden die Biokatalysen entsprechend der Zusammenstellung in Tabelle 9 durchgeführt. Im Anschluss an die Induktionsphase wurde das Substrat zugegeben und die Ansätze für weitere 24 h bei 28 °C und 150 Upm inkubiert. Die Proben wurden anschließend 2:1 mit Hexan extrahieren, die organische Phase in ein Probengefäß überführt und gaschromatographisch auf 12-Methyltridecansäure und 12-Methyltridecanal untersucht. Die Ergebnisse sind in **Tabelle 9** zusammengefasst:

**Tabelle 9**

| Versuchsergebnisse aus den Biokatalyseversuchen | | |
|---|---|---|
| **Substrat]** | **12-Methyltridecanal [µg/ml]** | **12-Methyltridecansäure [µg/ml]** |
| mit Lipase hydrolysierte Fettfraktion von *C*. *heteropsorus* (aus Beispiel 3) | 0,39 | 8,4 |
| Fettfraktion von *C. heterosporus* in DMSO gelöst, ohne vorherige Hydrolyse (aus Beispiel 2) | -- | 6,9 |
| 12-Methyltridecansäure | -- | 107,0 |
| Fettfraktion von *C. heterosporus* ohne vorherige Hydrolyse | 0,60 | 42,7 |

### BEISPIEL 12

### Chemische Umsetzung von Fettextrakten ex C. heterosporus zu 12-Methyltridecanal

Die zuvor gewonnenen Fettextrakte wurden in zwei Qualitäten untereilt: solche mit einem Fettgehalt von weniger als 1 g/L und solchen, mit höheren Gehalten. Die Umsetzung erfolgte in zwei Schritten, nämlich zunächst als eine Reduktion mit Hilfe Lithiumalanat und anschließend als Oxidation mit TEMPO/KBr/NaOCl. Die Reaktionsbedingungen sind in der folgenden **Tabelle 10** wiedergegeben:

**Tabelle 10**

| Reaktionsbedingungen* | | | | | |
|---|---|---|---|---|---|
| **Ansatz** | **Reaktionsbedingungen** | **Ausbeute** | | **GC-MS Reinheit [%]** | |
| | | **[g]** | **[%]** | **Direkt** | **Nach Kugelrohrdestillation** |
| 078 | LiAlH4 (0.7 eq.) RT, 3 h | 17,3 | 89 | 33,1 | 37.4 |
| 083 | LiAlH4 (0.7 eq.) RT, 3 h | 10,7 | 93 | 30,3 | 37.2 |
| 081 | KBr (0.28 eq.), TEMPO (0.067 eq.), NaClO (1.2 eq.), 0 °C, 4 h | 16,0 | 87 | 36,9 | 44,8 |
| 085 | KBr (0.30 eq.), TEMPO (0.073 eq.), NaClO (1.2 eq.), 0 °C, 3 h | | | 34,2 | |

| | | | | | |
|---|---|---|---|---|---|
| *) 078: 27.4 g of < 1g/L, 083: 17.8 g of >1g/L, 081: <1, 085: >1 | | | | | |

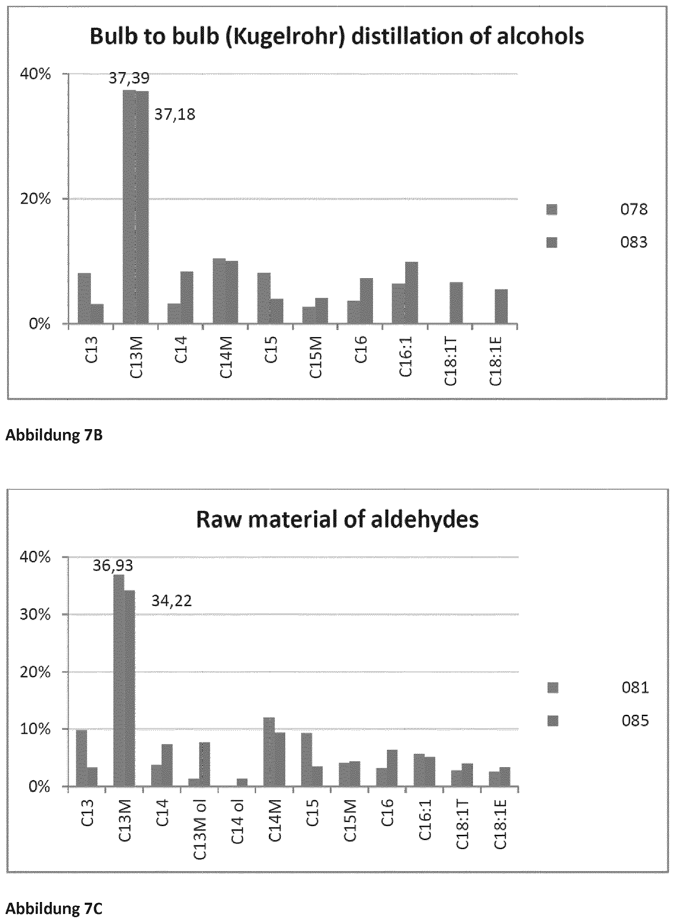

Die Reduktion mit Lithiumalanat ergab für das < 1 g/L-Experiment eine Ausbeute von 89 % und für das > 1g/L Experiment von 93 % (jeweils nach Destillation im Kugelrohr) bezogen auf den Gehalt an 12-Methyltridecansäure bzw. den entsprechenden Glyceriden. Der Doppelbindungsanteil wurde dabei von 22 % auf 6 % bzw. von 29 % auf 22 % vermindert. Die **Abbildungen 7A und 7B** geben für die beiden Ansätze mit der Bezeichnung 078 (< 1 g/L) bzw. 083 (> 1 g/L) die Zusammensetzung der resultierenden Alkohole vor und nach der Kugelrohrdestillation wieder.

Die nachfolgende Oxidation mit TEMPO ergab für den Fall < 1 g/L eine Ausbeute an Aldehyd von 97 % (081) und für den Fall > 1 g/L (085) 94 % bezogen auf den Gehalt an 12-Methyltridecansäure bzw. den entsprechenden Glyceriden (jeweils vor Destillation im Kugelrohr). Die Ergebnisse sind in **Abbildung 7C** wiedergegeben.

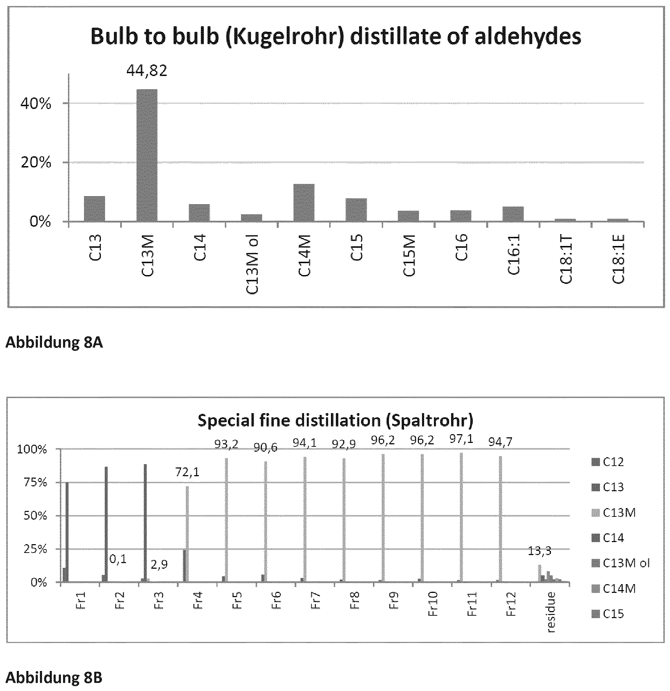

Die erhaltenen Aldehyd Fraktionen (#081 und #085) wurden vereinigt und wiederum im Kugelrohr destilliert. Es wurden 19,6 g (GC Reinheit 44.8 %) entsprechend einer Ausbeute von 77 % bezogen auf die Menge an 12-Methyltridecansäure bzw. den entsprechenden Glyceriden. Die Ergebnisse sind in **Abbildung 8A** wiedergegeben. Das resultierende Destillat wurde einer weiteren Aufreinigung im Spaltrohr unterworfen (72-83 °C, 0.5 mbar). Es wurden verschiedene Fraktionen mit Reinheiten von über 95 % erhalten **(****Abbildung 8B****).** Die Zusammensetzungen sind in **Tabelle 11** wiedergegeben.

**Tabelle 11**

| Reinheit der Fraktionen aus der Spaltrohrdestillation | | |
|---|---|---|
| | **Reinheit 12-MTD (GC)** | **Menge [g]** |
| 085 KD1 Fr1 | 44.8% | 19.6 |
| Spaltrohr Fr1 | - | 1.1 |
| Spaltrohr Fr2 | 0.1% | 0.1 |
| Spaltrohr Fr3 | 2.9% | 1.1 |
| Spaltrohr Fr4 | 72.1% | 1.1 |
| **Spaltrohr Fr5** | **93.2%** | **1.1** |
| **Spaltrohr Fr6** | **90.6%** | **0.2** |
| **Spaltrohr Fr7** | **94.1%** | **0.4** |
| **Spaltrohr Fr8** | **92.9%** | **0.3** |
| **Spaltrohr Fr9** | **96.2%** | **1.0** |
| **Spaltrohr Fr10** | **96.2%** | **0.4** |
| **Spaltrohr Fr11** | **97.1%** | **0.5** |
| **Spaltrohr Fr12** | **94.7%** | **0.2** |
| Rückstand | **13.3%** | 12.2 |

Die Gesamtausbeute für die Oxidation der beiden vereinigten Qualitäten aus den Fraktionen 5 bis 12 (4.1 g, Reinheit 94.7 %) betrug 38 % bezogen auf die Alkoholmischungen (17 g mit 37.4 % Reinheit und 10.5 g mit 37.2 % Reinheit).

Die Ausbeute an 12-Methytridecanal bezogen auf beide Schritte (Reduktion und Oxidation von 27.4 g < 1g/L Fettextrakt mit 30.4 GC-% 12-Methyltricansäuremethylester und 17.8 g > 1 g/L mit 27.1 GC-% Ester) betrug bezogen auf die Fraktionen 5 bis 12 (4.1 g, Reinheit 94.7 %) 34 %.

Ausgehend von der 95%-Reinheit der Fraktionen 5, 7 sowie 9 bis 12 (3.6 g) betrug die Ausbeute nach beiden Schritten 30 %.

## Patentansprüche

1. Verfahren zur Herstellung verzweigter Aldehyde umfassend die folgenden Schritte:
(a) Bereitstellen einer Kultur eines oder mehrerer Pilze der Gattung *Conidiobolus,* ausgewählt aus der *Spezies C. denaeosporus* oder *C. heterosporus,* und Erzeugung von Biomasse mit einem Gehalt an verzweigten Carbonsäuren in freier Form und/oder gebundener Form als Glyceride:
(b) Extraktion der Biomasse aus Schritt (a) zur Erzeugung eines ersten Zwischenproduktes enthaltend freie und/oder gebundene Carbonsäuren;
(c) optional chemische, enzymatische oder mikrobielle Hydrolyse der gebundenen Carbonsäuren aus dem ersten Zwischenprodukt;
und
(d) Behandlung des ersten Zwischenproduktes mit einem Reduktionsmittel chemischer Natur zur Überführung der freien und/oder gebundenen Carbonsäuren in die entsprechenden Alkohole sowie optionale Abtrennung eines oder mehrerer Alkohole von störenden Nebenkomponenten und Erzeugung des chemisch erzeugten zweiten Zwischenproduktes, welches diese Alkohole als Mischung oder in angereicherter Form enthält;
und
(f) Behandlung des chemisch erzeugten zweiten Zwischenproduktes mit einem Oxidationsmittel chemischer Natur zur Überführung der freien und/oder gebundenen Alkohole in die entsprechenden Aldehyde;
oder
(e) Behandlung des ersten Zwischenproduktes mit einem Reduktionsmittel biologischer Natur zur Überführung der freien und/oder gebundenen Carbonsäuren in die entsprechenden Aldehyde mit gleicher Anzahl an Kohlenstoffatomen im Vergleich zu den freien und/oder gebundenen Carbonsäuren oder in die korrespondierenden Aldehyde mit einer um eins verringerten Anzahl an Kohlenstoffatomen im Vergleich zu den freien und/oder gebundenen Carbonsäuren und Erzeugung des biologisch erzeugten zweiten Zwischenproduktes, welches diese Aldehyde enthält;
sowie gegebenenfalls
(g) Entfernung von störenden Nebenkomponenten aus den Fraktionen erhältlich nach den Schritten (d) und/oder (e) und/oder (f).

2. Verfahren nach Anspruch 1, **dadurch gekennzeichnet, dass** die verzweigten Aldehyde 12 bis 18 Kohlenstoffatome aufweisen.

3. Verfahren nach Anspruch 1 und/oder 2, **dadurch gekennzeichnet, dass** die verzweigten Aldehyde als Verzweigung eine Methylgruppe aufweisen.

4. Verfahren nach mindestens einem der Ansprüche 1 bis 3, **dadurch gekennzeichnet, dass** es sich bei den Aldehyden um 12-Methyltridecanal, 12-Methyltetradecanal, 14-Methylpentadecanal, 16-Methyloctadecanal oder deren Gemische handelt.

5. Verfahren nach mindestens einem der Ansprüche 1 bis 4, **dadurch gekennzeichnet, dass** man das erste Zwischenprodukt vor der Reduktion einer Hydrolyse unterwirft.

6. Verfahren nach mindestens einem der Ansprüche 1 bis 5, **dadurch gekennzeichnet, dass** man die Reduktion im Falle von Schritt (d) mit Lithiumalanat oder Natriumborhydrid durchführt.

7. Verfahren nach mindestens einem der Ansprüche 1 bis 5, **dadurch gekennzeichnet, dass** man die Reduktion im Falle von Schritt (e) unter Zuhilfenahme biologischer Systeme durchführt.

8. Verfahren nach dem Anspruch 7, **dadurch gekennzeichnet, dass** die Reduktion mit kommerziell erhältlicher Aldehyd-Dehydrogenasen (ALDH, EC 1.2.1.x) durchgeführt wird.

9. Verfahren nach dem Anspruch 7, **dadurch gekennzeichnet, dass** die Reduktion mit einer alpha-Dioxygenase durchgeführt wird, wobei hierbei gleichzeitig ein um ein C-Atom verkürztes Aldehyd entstehen kann.

10. Verfahren nach dem Anspruch 7, **dadurch gekennzeichnet, dass** die Reduktion mit aufgereinigtem Enzym Nsp-CAR und/oder MmFad9 durchgeführt wird

11. Verfahren nach dem Anspruch 7, **dadurch gekennzeichnet, dass** die Reduktion mit einem Ganzzellextrakt aus Nsp-CAR und/oder MmFad9 exprimierenden Zellen durchgeführt wird.

12. Verfahren nach dem Anspruch 7, **dadurch gekennzeichnet, dass** die Reduktion mit lebensfähigen Zellen durchgeführt wird, die in der Lage sind, die Enzyme Nsp-CAR und/oder MmFad9 zu exprimieren.

13. Verfahren nach mindestens einem der Ansprüche 1 bis 12, **dadurch gekennzeichnet, dass** man die Reduktion bei einer Temperatur im Bereich von 15 bis 25 °C durchführt.

14. Verfahren nach mindestens einem der Ansprüche 1 bis 13, **dadurch gekennzeichnet, dass** man in Schritt (f) als Oxidationsmittel TEMPO einsetzt.

15. Verfahren nach Anspruch 14, **dadurch gekennzeichnet, dass** man TEMPO zusammen mit einem Alkalibromid und einem Alkalihypochlorit einsetzt.

16. Verfahren nach Anspruch 15, **dadurch gekennzeichnet, dass** man TEMPO, Alkalibromid und Alkalihypochlorit im molaren Verhältnis von etwa 1:(2 bis 10):(10 bis 40) einsetzt.

17. Verfahren nach mindestens einem der Ansprüche 1 bis 16 **dadurch gekennzeichnet, dass** man die Oxidation bei einer Temperatur im Bereich von -5 bis +10 °C durchführt.

18. Verfahren nach mindestens einem der Ansprüche 1 bis 17, **dadurch gekennzeichnet, dass** man als weitere Schritte das Reduktionsprodukt, das Oxidationsprodukt oder beide einer destillativen Aufarbeitung unterwirft.

## Claims

1. Method for producing branched aldehydes, comprising the following steps:
(a) providing a culture of one or more fungi of the genus *Conidiobolus,* selected from the species *C. denaeosporus* or *C. heterosporus,* and production of biomass with an amount of branched carbonic acids in free form and/or bound form as glycerides:
(b) extraction of the biomass of step (a) for the production of a first intermediate product, containing free and/or bound carbonic acids;
(c) optionally chemical, enzymatic or microbial hydrolysis of the bound carbonic acids of the first intermediate product;
and
(d) treatment of the first intermediate product with a reducing agent of chemical nature for transmission of the free and/or bound carbonic acids into the respective alcohols as well as optional separation of one or further alcohols of disturbing side components and production of the chemically produced second intermediate product, which contains these alcohols as a mixture or in enriched form;
and
(f) treatment of the chemically produced second intermediate product with an oxidizing agent of chemical nature for transmission of the free and/or bound alcohols into the respective aldehydes;
or
(e) treatment of the first intermediate product with a reducing agent of biological nature for transmission of the free and/or bound carbonic acids into the respective aldehydes with the same number of carbon atoms compared to the free and/or bound carbonic acids or into the corresponding aldehydes with a number of carbon atoms reduced by one compared to the free and/or bound carbonic acids and production of the biologically produced second intermediate product, which contains these aldehydes;
as well as optionally
(g) removal of disturbing side components from the fractions obtainable after steps (d) and/or (e) and/or (f).

2. Method according to claim 1, **characterized in that** the branched aldehydes have 12 to 18 carbon atoms.

3. Method according to claim 1 and/or 2, **characterized in that** the branched aldehydes have a methyl group as branch.

4. Method according to at least one of claims 1 to 3, **characterized in that** the aldehydes are 12-methyltridecanal, 12-methyltetradecanal, 14-methylpentadecanal, 16-methyloctadecanal or their mixtures.

5. Method according to at least one of claims 1 to 4, **characterized in that** the first intermediate product is subjected to a hydrolysis before the reduction.

6. Method according to at least one of claims 1 to 5, **characterized in that** the reduction in case of step (d) is performed with lithium alanate or sodium boron hydride.

7. Method according to at least one of claims 1 to 5, **characterized in that** the reduction in case of step (e) is performed with the aid of biological systems.

8. Method according to claim 7, **characterized in that** the reduction is performed with commercially available aldehyde dehydrogenases (ALDH, EC 1,2.1x).

9. Method according to claim 7, **characterized in that** the reduction is performed with an alpha-dioxygenase, wherein an aldehyde shortened by one C-atom can simultaneously be produced herein.

10. Method according to claim 7, **characterized in that** the reduction is performed with purified enzyme Nsp-CAR and/or MmFad9.

11. Method according to claim 7, **characterized in that** the reduction is performed with a whole cell extract from Nsp-CAR and/or MmFad9 expressing cells.

12. Method according to claim 7, **characterized in that** the reduction is performed with viable cells, which are capable of expressing the enzymes Nsp-CAR and/or MmFad9.

13. Method according to at least one of claims 1 to 12, **characterized in that** the reduction is performed at a temperature in the range of from 15 to 25 °C.

14. Method according to at least one of the claims 1 to 13, **characterized in that** TEMPO is used as oxidizing agent in step (f).

15. Method according to claim 14, **characterized in that** TEMPO is used together with an alkali bromide and an alkali hypochlorite.

16. Method according to claim 15, **characterized in that** TEMPO, alkali bromide and alkali hypochlorite are used in a molar ratio of approximately 1:(2 to 10):(10 to 40).

17. Method according to at least one of claims 1 to 16, **characterized in that** the oxidation is performed at a temperature in a range of from -5 to +10 °C.

18. Method according to at least one of claims 1 to 17, **characterized in that** the reduction product, the oxidation product or both are subjected to a distillative processing as further steps.

## Revendications

1. Procédé de préparation d'aldéhydes ramifiés comprenant les étapes suivantes consistant à:
(a) fournir une culture d'un ou de plusieurs champignons du genre *Conidiobolus,* choisis parmi l'espèce *C. denaeosporus* ou *C. heterosporus,* et produire de la biomasse ayant une teneur en acides carboxyliques ramifiés sous forme libre et/ou sous forme liée en tant que glycérides;
(b) extraire la biomasse de l'étape (a) afin de produire un premier produit intermédiaire contenant des acides carboxyliques libres et/ou liés;
(c) en option une hydrolyse chimique, enzymatique ou microbienne des acides carboxyliques liés à partir du premier produit intermédiaire;
et
(d) traiter le premier produit intermédiaire avec un agent réducteur de nature chimique pour convertir les acides carboxyliques libres et/ou liés en alcools correspondants et séparer de façon optionnelle un ou plusieurs alcools de composants secondaires perturbateurs, et produire le deuxième produit intermédiaire produit chimiquement qui contient ces alcools en tant que mélange ou sous forme enrichie;
et
(f) traiter le deuxième produit intermédiaire produit chimiquement avec un agent oxydant de nature chimique afin de convertir les alcools libres et/ou liés en aldéhydes correspondants;
ou
(e) traiter le premier produit intermédiaire avec un agent réducteur de nature biologique pour convertir les acides carboxyliques libres et/ou liés en aldéhydes correspondants ayant le même nombre d'atomes de carbone par rapport aux acides carboxyliques libres et/ou liés, ou en aldéhydes correspondants ayant un nombre d'atomes de carbone réduit d'un par rapport aux acides carboxyliques libres et/ou liés, et produire le deuxième produit intermédiaire produit biologiquement qui contient ces aldéhydes;
ainsi que, le cas échéant,
(g) éliminer des composants secondaires perturbateurs des fractions qui peuvent être obtenues selon les étapes (d) et/ou (e) et/ou (f).

2. Procédé selon la revendication 1, **caractérisé par le fait que** les aldéhydes ramifiés présentent entre 12 et 18 atomes de carbone.

3. Procédé selon la revendication 1 et/ou 2, **caractérisé par le fait que** les aldéhydes ramifiés présentent un groupe méthyle en tant que ramification.

4. Procédé selon l'une au moins des revendications 1 à 3, **caractérisé par le fait que** les aldéhydes sont le 12-méthyltridécanal, le 12-méthyltétradécanal, le 14-méthyl-pentadécanal, le 16-méthyloctadécanal ou leurs mélanges.

5. Procédé selon l'une au moins des revendications 1 à 4, **caractérisé par le fait que** le premier produit intermédiaire est soumis à une hydrolyse avant la réduction.

6. Procédé selon l'une au moins des revendications 1 à 5, **caractérisé par le fait que**, dans le cas de l'étape (d), la réduction est réalisée avec de l'alanate de lithium ou du borohydrure de sodium.

7. Procédé selon l'une au moins des revendications 1 à 5, **caractérisé par le fait que**, dans le cas de l'étape (e), la réduction est réalisée à l'aide de systèmes biologiques.

8. Procédé selon la revendication 7, **caractérisé par le fait que** la réduction est réalisée avec de l'aldéhyde déshydrogénase disponible dans le commerce (ALDH, EC 1.2.1.x).

9. Procédé selon la revendication 7, **caractérisé par le fait que** la réduction est réalisée avec une alpha-dioxygénase, un aldéhyde raccourci d'un atome de carbone y étant formé en même temps.

10. Procédé selon la revendication 7, **caractérisé par** le fait la réduction est réalisée avec de l'enzyme purifiée Nsp-CAR et/ou MmFad9.

11. Procédé selon la revendication 7, **caractérisé par le fait que** la réduction est réalisée avec un extrait de cellules entières à partir de cellules exprimant Nsp-CAR et/ou MmFad9.

12. Procédé selon la revendication 7, **caractérisé par le fait que** la réduction est réalisée avec des cellules viables qui sont capables d'exprimer les enzymes Nsp-CAR et/ou MmFad9.

13. Procédé selon l'une au moins des revendications 1 à 12, **caractérisé par le fait que** la réduction est réalisée à une température comprise entre 15 et 25 °C.

14. Procédé selon l'une au moins des revendications 1 à 13, **caractérisé par le fait que** le TEMPO est utilisé comme agent d'oxydation à l'étape (f).

15. Procédé selon la revendication 14, **caractérisé par le fait que** l'on utilise le TEMPO conjointement avec un bromure alcalin et un hypochlorite alcalin.

16. Procédé selon la revendication 15, **caractérisé par le fait que** l'on utilise le TEMPO, du bromure alcalin et de l'hypochlorite alcalin dans un rapport molaire compris entre environ 1 : (2 à 10) : (10 à 40).

17. Procédé selon l'une au moins des revendications 1 à 16, **caractérisé par le fait que** l'oxydation est réalisée à une température comprise entre -5 et +10 °C.

18. Procédé selon l'une au moins des revendications 1 à 17, **caractérisé par le fait que**, comme autres étapes, le produit de réduction, le produit d'oxydation ou les deux est/sont soumis à un retraitement par distillation.
